# FASCICULE DE BREVET EUROPEEN

(11) **EP 0 340 079 B1**
(45) Date de publication et mention de la délivrance du brevet: **03.02.1993**
(21) Numéro de dépôt: 89401097.4
(22) Date de dépôt: 19.04.1989
(51) Int. Cl.: B01F 17/00, C10M 173/00, B01J 13/00, A61K 47/00, A61K 9/00

(54) **Matériaux viscoélastiques isotropes à base d'eau, de tensioactifs fluorés et d'huiles fluorées, leur procédé d'obtention et leurs applications dans divers domaines, tels que ceux de l'optique, de la pharmacologie et de l'électrodynamique**
Viskoelastisches isotropes Material auf Basis von Wasser, fluorierten oberflächenaktiven Substanzen und fluorierten Ölen, ihr Herstellungsprozess und ihre Anwendungen auf verschiedenen Gebieten, wie beispielsweise die der Optik, der Pharmakologie und der Elektrodynamik
Viscoelastic isotropic material based on water, fluorinated surfactants, and fluorinated oils, their preparation and their use in different fields, such as the optical, pharmacological and electrodynamical field

(30) Priorité: 20.04.1988 FR 8805229
(43) Date de publication de la demande: 02.11.1989
(73) Titulaire: ELF SANOFI, 75008 Paris (FR)
(72) Inventeur: Delpuech, Jean-Jacques, F-54620 Laxou (FR); Matos, Louis, Pointe-Noire (CG); Mouni, El Mostafa C.U. du Placieux, F-54600 Villers-lès-Nancy (FR); Ravey, Jean-Claude, F-54850 Messein (FR); Selve, Claude, F-54600 Villers-lès-Nancy (FR); Serratrice, Guy, F-38190 Crolles (FR); Stebe, Marie-José, F-54500 Vandoeuvre-lès-Nancy (FR); Cambon, Aimé, F-06000 Nice (FR); Thiollet, Gérard, F-91590 Cerny (FR)
(74) Mandataire: Moncheny, Michel

(56) Documents cités:
- EP-A- 0 051 526
- FR-A- 2 249 657
- GB-A- 2 087 882
- CHEMICAL ABSTRACTS, vol. 83, no. 1, 14 juillet 1975, page 349, abstract no. 15661d, Columbus, Ohio, US
- JOURNAL OF COLLOID AND INTERFACE SCIENCE, vol. 98, no. 2, avril 1984, pages 515-522, Academic Press, Inc.; A. ROBERT et al.: "Solubilization of water in binary mixtures of fluorocarbons and nonionic fluorinated sufactants: existence domains of reverse microemulsions"
- JOURNAL OF THE AMERICAN CHEMICAL SOCIETY, vol. 106, 1984, pages 6162-6171, American Chemical Society; G. MATHIS et al.: "A novel class of nonionic microemulsions: fluorocarbons in aqueous solutions of fluorinated poly(oxyethylene) surfactants"

## Description

La présente invention concerne des matériaux isotropes viscoélastiques transparents à base de tensioactifs fluorés et d'huiles fluorées renfermant une très forte proportion d'eau de l'ordre de 60 à 98 % en poids.

On entend dans la présente description par "matériaux viscoélastiques" des matériaux qui sont à la fois visqueux et élastiques et qui ont l'aspect et la consistance de "gels" ; c'est du reste ce terme qui sera utilisé par la suite pour les désigner pour une raison de commodité.

De façon plus précise, les tensioactifs fluorés entrant dans ces matériaux sont des tensioactifs non ioniques à chaîne hydrophobe essentiellement fluorée. Ils seront définis ultérieurement, de même que les huiles fluorées.

Bien que présentant une très forte teneur en eau, les gels selon l'invention appartiennent à une catégorie tout à fait particulière, se distinguant des gels qu'il est convenu d'appeler des hydrogels.

En effet, on connaît différentes classes d'hydrogels selon la structure chimique ou physique de ces derniers. Une classification souvent utilisée, en particulier dans le domaine biomédical, distingue les hydrogels neutres ou non ioniques (du type cellulose, par exemple), les hydrogels ioniques (du type silicates, par exemple) et les réseaux polymériques interpénétrés (IPN). Ces hydrogels connus présentent généralement des réseaux gonflés d'eau (structures pontées) de polymères ou de copolymères hydrophiles. Ces réseaux sont tridimensionnels et les pontages sont formés par des liaisons covalentes ou ioniques. Fréquemment, des liaisons plus faibles du type Van der Waals, ou hydrogène, peuvent servir de ponts formant ainsi des réseaux gonflés qui se comportent comme des hydrogels.

Enfin, des polymères hydrophiles semi-cristallins et non pontés peuvent former des hydrogels en gonflant puisque les cristallites se comportent comme des ponts physiques, étant donné qu'ils ne se dissolvent pas dans l'eau, contrairement aux parties amorphes.

EP-A-0051526 décrit des microémulsions de type huile dans eau à base de fluocarbures et d'agent tensioactif fluoré non ionique capables de transporter l'oxygène.

Les gels selon la présente invention se différencient des hydrogels existants par leur structure qui est une structure "compartimentée", c'est-à-dire une structure qui, observée au microscope électronique, présente une très forte densité de globules d'eau entourés d'une fine membrane de tensioactif et inclus dans une phase continue huile/ tensioactif. Les propriétés du ou des tensioactif(s) faisant partie de cette structure influent sur le comportement des "gels" résultants vis-à- vis de l'eau et des huiles puisqu'ils présentent finalement une nette imperméabilité à l'eau et une perméabilité aux composés hydrophobes solubles dans les fluoro-carbures, notamment les gaz (par exemple gaz respiratoires) et les composés fluorés ou même hydrogénés pas trop fluophobes. Un tel résultat s'obtient avec des tensio-actifs non ioniques à partie hydrophobe essentiellement fluorée et d'hydrophilie relativement peu élevée et donc présentant avantageusement une HLB appropriée calculée selon la formule de Griffin inférieure à 8 et de préférence comprise entre 4 et 6.

La présente demande a donc pour objet des matériaux isotropes viscoélastiques à base d'eau, de tensio-actifs fluorés et d'huiles fluorées ayant l'aspect et la consistance de gels, caractérisés en ce qu'ils renferment une très forte proportion d'eau, de l'ordre de 60 à 98 % en poids et que leur structure est une structure compartimentée microcellulaire présentant une très forte densité de globules d'eau entourés d'une fine membrane de tensioactif et inclus dans une phase continue huile/tensioactif, cette structure étant imperméable à l'eau et perméable aux composés hydrophobes solubles dans les fluorocarbures.

Suivant d'autres caractéristiques :
- les globules d'eau entrant dans la structure des matériaux de l'invention ont une taille moyenne environ de l'ordre du micron ;
- les matériaux, préparés avec de l'eau salée, présentent une conductivité électrique très basse, de l'ordre de quelques dizièmes de micro-siemens/cm.

Les matériaux isotropes viscoélastiques selon l'invention se caractérisent également par la combinaison des autres propriétés physiques suivantes : transparence, caractère tensioactif, inertie chimique, stabilité, propriétés de perméation.

Des matériaux préférés sont ceux caractérisés en ce que les tensioactifs fluorés sont des tensioactifs non ioniques à chaîne essentiellement fluorée et que les huiles fluorées sont choisies parmi les hydrocarbures totalement ou partiellement fluorés comportant au moins 6 atomes de carbone, de préférence de 6 à 20 atomes de carbone et en particulier environ 10 atomes de carbone.

A titre de tensioactifs à chaîne hydrophobe essentiellement fluorée utilisables dans l'invention, on citera en particulier
- les produits de la forme :

   RF(CH2)p R(OH) (Famille I)

   où p est généralement inférieur à 3, dans lesquels RF est une chaîne perfluorée, R désigne un enchaînement de motifs oxyéthylènes (OC2H4), où l'un ou plusieurs des atomes d'oxygène peuvent être remplacés par des atomes de soufre.
   Des produits de cette famille avec p = 2 sont notamment décrits dans le FR-A-2.565.226.
- les produits de la forme : 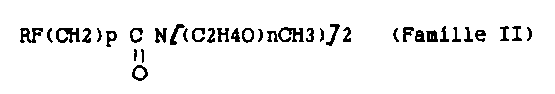 où p est généralement inférieur à 3.
   Des produits de cette famille avec p = 1 ou p = 0 sont décrits dans la demande de brevet français N° 87.06515 ;
- ainsi que tous les produits tensioactifs a chaîne(s) fluorée(s) présentant une hydrophilie relativement peu élevée, caractérisée par une HLB calculée selon la formule de Griffin inférieure à 8, associant une partie hydrophile comprenant des groupements polyoxyéthylène -(OC2H4)n-, avec n > 2, à une partie hydrophobe fluorée comprenant des motifs -CxF2x- avec 4 < x < 12.

Comme huile fluorée entrant dans les mélanges ternaires selon l'invention, on citera en particulier des carbures totalement ou partiellement fluorés comportant typiquement 6 à 20 atomes de carbone, en particulier des carbures présentant 10 atomes de carbone, pouvant comporter des ramifications, des insaturations, des cycles aliphatiques ou aromatiques et des hétéro-atomes.

Il est à noter qu'un mélange direct des trois composants dans les proportions correspondant à la composition finale d'un gel ne conduit pas nécessairement à l'obtention aisée de ce gel. Le prémélange préalable avec passage par le stade d'un milieu limpide isotrope dont on augmente ensuite de manière inattendue la viscosité par addition progressive d'eau, est une variante essentielle du procédé d'obtention.

Des gels selon l'invention pourront également être obtenus en remplaçant l'eau par un mélange d'eau et d'un solvant miscible à l'eau, par exemple par un mélange eau-DMSO ou eau-propylèneglycol, ou par une solution aqueuse homogène ayant dissous un ou plusieurs solutés, par exemple une solution ionique ou un milieu de culture cellulaire, ou une substance polymérisable, par exemple un acrylate.

De même, le gel selon l'invention pourra contenir dans l'une ou l'autre de ses phases (eau ou huile), ou même dans les deux, toute substance désirée pouvant avoir une action propre dans la ou les applications que l'on destine au gel qui la contient.

Ainsi, la présente invention couvre aussi ces applications, lesquelles sont, comme on le comprendra, directement liées aux propriétés physiques des produits en cause, à savoir :
- transparence des gels obtenus, notamment de ceux obtenus entre l'eau, l'huile fluorée et le tensio-actif non ionique ; en effet, l'indice de réfraction des produits étant voisin de celui de l'eau, les hydrogels obtenus sont parfaitement transparents, propriété essentielle pour des applications optiques ou photochimiques;
- viscoélasticité qui peut être modulée en fonction de la proportion des constituants : ces produits se présentent en effet sous forme d'une gelée facilement manipulable.

Leur viscosité apparente peut varier dans de grandes limites en jouant sur la quantité d'eau incorporée, la nature du tensioactif et de l'huile, et la température. Des gels de grande rigidité sont obtenus pour de fortes concentrations en eau (90-98 % en poids), tandis que des gels relativement coulants sont obtenus à concentration en eau plus faible (70-90 %), les rendant ainsi plus aptes à être introduits dans des cavités comme matière de remplissage. Leur comportement rhéologique est du type "plastique", c'est-à-dire que leur étalement nécessite l'application d'une contrainte minimale supérieure à un seuil qui dépend de la composition du gel. A titre d'exemple de ce seuil, pour un gel à base de perfluorodécaline, de C6F13C2H4S(C2H4O)3 H et d'eau est d'environ 16 N/m2 lorsque le gel contient 80 % d'eau, d'environ 35 à 40 N/m2 lorsque le gel contient 90% d'eau ;
- caractère tensioactif : les manipulations précédentes sont encore facilitées par le fait que ces gels ont un pouvoir mouillant élevé des surfaces car constitués à partir de tensioactifs fluorés, propriété intéressante pour de nombreuses applications en formulation (cosmétiques par exemple). Ces tensioactifs fluorés apportent en effet leur pouvoir de glisse et d'étalement exceptionnel, recherché dans de nombreuses applications (lubrification, film, textiles...);
- inertie chimique : la haute teneur en fluor confère aux produits entrant dans la composition des gels, un caractère d'inaltérabilité : stabilité thermique notamment jusqu'à 100°C, en particulier de 10 à 60°C, résistance à l'oxydation notamment aux oxydants forts comme le permanganate de potassium ou sel de chrome, à l'hydrolyse et à l'action des agents chimiques ou biochimiques usuels tels que des acides comme l'acide citrique, l'acide nitrique, l'acide chlorhydrique et en général les hydracides, des bases comme la potasse ou la soude et les bases fortes ou faibles en général, les produits ne sont pas des substrats d'enzymes et en particulier ne sont pas des substrats des protéases ;

stabilité : la stabilité des gels de la présente invention varie en sens inverse de la quantité d'eau présente dans le mélange. Ainsi, pour des fractions massiques en eau inférieures à 90-92 %, les gels sont très stables : au bout de quelques jours seulement, apparaît une fine pellicule d'eau à la surface du gel sans qu'il perde sa consistance initiale. Par contre, lorsque la quantité d'eau atteint 98 %, il apparaît plus rapidement une couche d'eau qui tend à augmenter avec le temps.

Cependant, la couche d'eau exsudée est réincorporable par simple agitation, redonnant au gel sa consistance initiale.

La stabilité varie, bien entendu, avec la température et la teneur en eau. Ces gels peuvent être additionnés de quantités modérées (de l'ordre de la mole par litre) d'électrolytes ou de solutés moléculaires soit dans la phase aqueuse, soit dans la phase constituant les parois des compartiments (fluorocarbure + tensioactif) sans être détruits : leur zone d'existence est simplement décalée en température.
- Imperméabilité notamment à l'eau et perméabilité, particulièrement aux huiles fluorées.

La présente invention concerne également un procédé d'obtention des matériaux viscoélastiques en question.

Ce procédé consiste, en particulier, à mettre en présence l'huile fluorée, le tensioactif fluoré et la quantité nécessaire et suffisante d'eau pour obtenir un pré-mélange limpide isotrope puis à rajouter progressivement de l'eau jusqu'à obtention du gel désiré.

Pour obtenir la zone de pré-mélange limpide isotrope, on peut par exemple établir le diagramme ternaire des composants choisis.

Il y a une très grande souplesse de formulation permettant à volonté l'obtention de gels transparents et stables pour une zone de température quelconque comprise entre la température ambiante et 80° C, en jouant, de manière combinée, sur l'hydrophilie du surfactif, la présence de solutés dans l'eau, le rapport massique fluorocarbure/tensioactif et la nature de l'huile. En particulier, une augmentation de température a le même effet qu'un abaissement de HLB. L'hydrophilie du surfactif peut être réglée à volonté soit en changeant le tensioactif, soit en effectuant des mélanges de tensioactifs de HLB différentes. Un dernier moyen, plus subtil, de changer la zone de température consiste à varier la composition isotopique de l'eau :
une augmentation de température pouvant atteindre 20 ° C est observée en substituant l'eau lourde (D20) à l'eau légère (H2O) des variations intermédiaires étant observées en utilisant des mélanges H2O/D2O de rapport isotopique variable.

Enfin, la structure compartimentée tout à fait originale de ces hydrogels, différente en particulier de celle de hydrogels connus jusqu'à présent, permet d'envisager des applications différentes : il est ainsi possible de réaliser des milieux ioniques aqueux conduisant faiblement le courant, donc des diélectriques très spéciaux à forte polarisation. Le compartimentage permet la réalisation par mélanges, de gels mixtes chargés avec des ions habituellement incompatibles entre eux, en raison de phénomènes soit de précipitation, soit d'oxydoréduction.

De manière générale, cette structure compartimentée peut permettre la réalisation de réactions chimiques en milieu confiné. Un exemple classique est l'obtention de matériaux solides divisés, pulvérulents ou au contraire sous forme de mousses, par polymérisation photochimique in situ d'un monomère hydro ou fluosoluble.

Des propriétés d'encapsulation, analogues à celles des vésicules (avec moins de stabilité mais inversement plus de facilité d'obtention et de réversibilité), sont prévisibles pour de tels systèmes, entraînant de nombreuses applications ou formulations, notamment en pharmacologie.

Pour les mêmes raisons, des propriétés de perméation résulteront de cette structure compartimentée : imperméabilité à l'eau et aux solutions ioniques/aqueuses et perméabilité aux composés hydrophobes solubles dans les fluorocarbures, notamment les gaz (par exemple les gaz respiratoires) et les composés fluorés, ou même hydrogénés pas trop fluophobes. Des applications potentielles de ces propriétés de perméation concernent donc le domaine de la chromatographie sous toutes ses formes, la réalisation de membranes semi-perméables inertes chimiquement, l'utilisation comme support en pharmacie (pommades cicatrisantes car perméables à l'oxygène et au gaz carbonique) ou en ophtalmologie.

La présente demande a enfin pour objet l'application des matériaux tels que définis ci-dessus, notamment dans le domaine de l'optique, des matériaux de remplissage, des cosmétiques, de la pharmacologie, des lubrifiants, des textiles, de la photochimie et de l'électrodynamique.

Les exemples suivants sont donnés à titre illustratif et non limitatif de la présente invention.

### 1. Exemples de préparation des gels selon l'invention

### Mode opératoire général

On réalise une solution constituée d'un quantité définie du tensioactif (n) et d'une quantité définie de l'huile perfluorée choisie (p).

A cette solution, on additionne la solution aqueuse par quantités définies (m) en homogénéisant par agitation et chauffage jusqu'à atteindre les proportions choisies.

### Exemple 1 :

72,1 % eau - 5,2 % surfactif et 22,7 % perfluorodécaline : à une solution de n = 10,4 mg de C₁₀F₂₁CH₂C(O)N[(C₂H₄O)₂Me]₂ et de p = 45,4 mg de perfluorodécaline, on additionne suivant le mode opératoire décrit ci-dessus m = 144,2 mg d'eau. Le gel obtenu est stable à partir de 10°C et reste stable à 60°C.

### Exemple 2 :

81 % eau - 4 % surfactif et 15 % de perfluorodécaline : à une solution de n = 16 mg de C₁₀F₂₁CH₂C(O)N[(C₂H₄O)₂Me]₂ et p = 60 mg de perfluorodécaline, on additionne suivant le mode opératoire général décrit ci-dessus m = 324 mg d'eau. Le gel obtenu est stable dès 10°C et reste stable à 60°C.

### Exemple 3 :

97 % eau - 0,7 % surfactif et 2,3 % perfluorodécaline : à une solution n = 7 mg de C₁₀F₂₁CH₂C(O)N[C₂H₄O)₂Me]₂ et p = 23 mg de perfluorodécaline, on additionne suivant le mode opératoire général décrit ci-dessus m = 970 mg. d'eau. Le gel obtenu est stable au-delà de 10°C et reste stable à 60°C.

### Exemple 4 :

97 % eau - 0,6 % surfactif - 2,4 % perfluorodécaline : à une solution de n = 6 mg de C₆F₁₃C₂H₄S(C₂H₄O)₃H et p = 24 mg de perfluorodécaline on additionne suivant le mode opératoire général décrit ci-dessus m = 970 mg d'eau. Le gel obtenu est transparent et stable entre 20° et 30°C.

### Exemple 5 :

90 % eau - 4 % surfactif - 6 % C₄F₉CH=CHC₄F₉: à une solution de n = 40 mg de C₆F₁₃C₂H₄S(C₂H₄O)₃H et p = 60 mg de C₄F₉CH=CHC₄F₉, on additionne suivant le mode opératoire général décrit ci-dessus m = 900 mg d'eau. Le gel obtenu est transparent et stable entre 20° et 30°C.

### Exemple 6 :

90 % eau - 2,3%surfactif - 7,7% C₈F₁₇CH=CH₂ : à une solution de n = 23 mg de C₆F₁₃C₂H₄S(C₂H₄O)₂ C₂H₄S(C₂H₄O)₃H et p = 77 mg de C₈F₁₇CH=CH₂, on ajoute m = 900 mg d'eau. Le gel obtenu est transparent et stable entre 20° et 30°C.

### Exemple 7 :

90 % eau - 3,5 % surfactif - 6,5 % C₈F₁₇CH=CH₂: à une solution de n = 35 mg de C₆F₁₃C₂H₄S(C₂H₄O)₃H et p = 65 mg de C₈F₁₇CH=CH₂, on ajoute m = 900 mg d'eau. Le gel obtenu est transparent et stable entre 20° et 30°C.

### Exemple 8 :

81 % eau - 4 % surfactif - 15 % C₈F₁₇CH=CH₂: à une solution de n = 40 mg de C₆F₁₃C₂H₄SC₂H₄OC₂H₄S(C₂H₄O)₃H et p = 150 mg de C₈F₁₇CH=CH₂, on ajoute m = 810 mg d'eau. Le gel obtenu est transparent et stable entre 20° et 30°C.

### Exemple 9 :

88 % eau - 4 % surfactif - 8 % C₈F₁₇CH=CH₂: à une solution de n = 40 mg de C₆F₁₃C₂H₄S(C₂H₄O)₄H et p = 80 mg de C₈F₁₇CH=CH₂, on ajoute m = 880 mg d'eau. Le gel obtenu est transparent et stable entre 20° et 30°C.

### Exemple 10 :

90 % eau - 1,8 % surfactif - 8,2 % perfluorodécaline : à une solution de n = 18 mg de C₆F₁₃C₂H₄S(C₂H₄O)₄H et p = 82 mg de perfluorodécaline, on ajoute m = 900 mg d'eau. Le gel obtenu est transparent et stable entre 20° et 30°C.

### Exemple 11 :

85,8 % eau - 4,4 % tensioactif - 9,3 % perfluorodécaline: à une solution de 44,0 mg de C₆F₁₃CH₂(OCH₂CH₂)₃OH et de 82,5 mg de perfluorodécaline, on additionne 764,0 mg d'eau. Le gel obtenu est transparent et stable entre environ 15° et 28°C.

### Exemple 12 :

74,0 % eau - 8,5 % tensioactif - 17,5 % perfluorodécaline : à une solution de 50,0 mg de C₆F₁₃CH₂(OCH₂CH₂)₃OH et de 102,5 mg de perfluorodécaline, on additionne 434,0 mg d'eau. Le gel obtenu est transparent et stable entre environ 20 et 30°C.

### Exemple 13 :

71,7 % eau - 12,1 % tensioactif - 16,2 % perfluorodécaline : à une solution de 72,6 mg de C₆F₁₃CH₂(OCH₂CH₂)₃OH et de 97,2 mg de perfluorodécaline, on additionne 430,2 mg. d'eau. Le gel obtenu est transparent et stable aux environs de 40°C.

### Exemple 14 :

54,1 % eau - 13,7 % tensio-actif - 32,1 % perfluorodécaline : à une solution de 41,8 mg de C₆F₁₃CH₂(OCH₂CH₂)₄OH et de 98,0 mg de perfluorodécaline, on additionne 165,1 mg d'eau. Le gel obtenu est stable et transparent à 50°C.

### Exemple 15 :

87,3 % eau - 3,8 % tensio-actif - 8,9 % huile : à une solution de 11,4 mg de C₆F₁₃CH₂(OCH₂CH₂)₄OH et de 26,7 mg de perfluorodécaline, on additionne 261,9 mg d'eau. Le gel obtenu est transparent et stable à 38°C.

### Exemple 16 -

81 % eau/DMSO (v/v), 3 % surfactif et 16 % perfluorodécaline : à une solution de n = 30 mg de C1OF21CH2C(O)N/(C2H4O)2Me/2 et de p = 160 mg de perfluorodécaline, on additionne, suivant le mode opératoire général décrit ci-dessus, m = 810 mg de phase aqueuse /constituée par une solution de 1 volume d'eau, 1 volume de diméthylsulfoxyde (DMSO)/. Le gel obtenu est stable dès 10°C et reste stable à 40° C.

### Exemple 17 -

81 % eau-propylèneglycol (v/v), 3 % surfactif et 16 % perfluorodécaline : la même composition que celle de l'exemple 16 est préparée selon le mode opératoire général, en utilisant comme phase aqueuse une solution constituée par 1 volume d'eau et 1 volume de propylène-glycol. Le gel obtenu présente le même stabilité que celui de l'exemple 16.

### Exemple 18 -

Le gel de composition identique à celui de l'exemple 4, mais préparé avec une phase aqueuse constituée d'eau dans laquelle on a dissous 10 g/litre de NaCl, a une zone de température de stabilité optimale, qui va de 15° à 25° C. (On constate un abaissement de 4°C pour la zone de stabilité optimale).

### Exemple 19 -

Le gel de composition identique à celui de l'exemple 4, mais préparé avec de l'eau lourde (D2O à la place de H2O) a une zone de température de stabilité optimale qui va de 45° à 55° C. (On constate un décalage de + 25°C pour la zone de stabilité optimale).

### Exemple 20 -

Le gel de composition identique à celui de l'exemple 4 mais préparé avec une phase aqueuse constituée par une solution de 60 g de chlorure de sodium (NaCl) dans l'eau lourde (D2O) a une zone de température de stabilité optimale, identique à celle du gel de l'exemple 4.

### Exemple 21 -

77 % eau, 5 % tensioactifs (mélange artificiel et précis de deux surfactifs), 18 % de perfluorodécaline : à une solution de n = n1+n2, n1=12,5 mg de C6F13C2H4S(C2H4O)3H et de n2 = 12,5 mg de C6F13C2H4S(C2H4O)4H, on additionne p = 90 mg de perfluorodécaline et m = 385 mg d'eau. Le gel obtenu suivant le mode opératoire général est stable entre 25° et 35° C.

### Exemple 22 -

97,5 % eau, 1,7 % C8F17CH=CH2, 0,8 % surfactif : à une solution de 1 g de C6F13C2H4S(C2H4O)3H et 2,1 g de C8F17CH=CH2, on ajoute 120 g d'eau. Le gel obtenu est transparent et stable entre 18° et 30° C.

### Exemple 23 -

96,1 % eau, 2,68 % C8F17CH=CH2, 1,28 % surfactif : à une solution de 1 g de C6F13C2H4S(C2H4O)3H et 2,1 g de C8F17CH=CH2, on ajoute 75 g d'eau. Le gel obtenu est transparent et stable entre 18° et 30° C.

### Exemple 24 -

94,16 % eau, 3,95 % C8F17CH=CH2, 1,88 % surfactif : à une solution de 1 g de C6F13C2H4S(C2H4O)3H et 2,1 g de C8F17CH=CH2, on ajoute 50 g d'eau. Le gel obtenu est transparent et stable entre 18° et 30° C.

### Exemple 25 -

90,6 % eau, 6,35 % C8F17CH=CH2, 3,02 % surfactif : à une solution de 1 g de C6F13C2H4S(C2H4O)3H et 2,1 g de C8F17CH=CH2, on ajoute 30 g d'eau. Le gel obtenu est transparent et stable entre 18° et 30° C.

### 2. Expériences illustrant quelques propriétés des gels

### Diffusivité entre gel et eau :

Sur les gels de l'exemple 3 (97 % d'eau, 0,7 % de surfactif et 2,3 % de perfluorodécaline) et 4 (97 % d'eau, 0,6 % de surfactif et 2,4 % de perfluorodécaline), on dépose une solution aqueuse colorée de bromophénol (coloration rouge-rosé) et on abandonne (sans agiter) à 25° C ; après 8 jours, on ne constate aucune coloration de la partie fluogel.

La même expérience, réalisée en remplaçant les gels selon l'invention par un gel aqueux d'agar-agar montre une coloration complété du gel au bout de 24 heures sur 2 cm d'épaisseur.

Diffusivité entre gel et huile fluorée
(a) Sur 1 ml de gel constitué suivant l'exemple 2 (81 % d'eau, 4 % de C10F21CH2CON/(C2H40)2Me/2, 15% de perfluorodécaline), on "dépose" environ 1 ml de perfluorodécaline saturée en iode. Après environ deux heures au repos, le gel est coloré en jaune par l'iode.
(b) Si la perfluorodécaline, saturée en iode, est déposée sur le gel, les densités respectives de la phase perfluorodécaline et gel étant dpF > dgel, on constate que le gel se "découpe" en morceaux qui "traversent" la phase perfluorodécaline et finalement la phase gel "passe" totalement au-dessus sans changement apparent des quantités respectives de gel et de perfluorodécaline ; là encore, le gel se colore en jaune.
(c) Ces mêmes observations ont été faites avec les gels de l'exemple 10 et de l'exemple 12.

Conductivité

Une mesure a été réalisée sur un mélange composé de 74 % d'eau (additionnée de NaCl à 0,5 g/l), 9 % de surfactif C6F13C2H4S(C2H40)3H et de 17 % de C8F17CH=CH2.

La conductivité spécifique est alors de 0,46uS.cm-1, valeur très faible en comparaison de celle de l'eau salée à 0,5 g/l (946uS.cm-1 à 25° C).

## Revendications (Revendications pour l'(les) Etat(s) contractant(s) suivant(s): AT, BE, CH, DE, FR, GB, GR, IT, LI, LU, NL, SE)

1. Matériaux isotropes viscoélastiques à base d'eau, de tensioactifs fluorés et d'huiles fluorées ayant l'aspect et la consistance de gels, caractérisés en ce qu'ils renferment de 60 à 98 % en poids d'eau ou d'un mélange eau-solvant miscible à l'eau et que leur structure est une structure compartimentée microcellulaire, présentant des globules d'eau ou de mélange entourés d'une membrane de tensioactif et inclus dans une phase continue huile/tensioactif, cette structure étant imperméable à l'eau et perméable aux composés hydrophobes solubles dans les fluocarbures.

2. Matériaux selon la revendication 1, caractérisés en ce que le tensioactif est choisi parmi les tensioactifs non ioniques à chaîne(s) fluorée(s) présentant une hydrophilie caractérisée par une HBL calculée selon la formule de Griffin inférieure à 8.

3. Matériaux selon la revendication 2, caractérisés en ce que le tensioactif associe une partie hydrophile comprenant des groupements polyoxyéthylènes -(OC₂H₄)n- avec n > 2, à une partie hydrophobe fluorée comprenant des motifs -CₓF2ₓ-, avec 4 < x < 12.

4. Matériaux selon l'une des revendications 1 à 3, caractérisés en ce que les globules d'eau ou de mélange ont une taille moyenne de l'ordre du micron.

5. Matériaux selon l'une quelconque des revendications 1 à 4, caractérisés en ce que les tensioactifs fluorés sont des tensioactifs non ioniques à chaîne essentiellement fluorée et que les huiles fluorées sont choisies parmi les carbures totalement ou partiellement fluorés comportant au moins 6 atomes de carbone.

6. Matériaux selon la revendication 5, caractérisés en ce que les tensioactifs à chaîne essentiellement fluorée sont choisis parmi :
- les produits de la forme :
RF(CH2)ₚR(OH) (famille I)
où p est inférieur à 3, dans lesquels RF est une chaîne perfluorée,
R désigne un enchaînement de motifs oxyéthylènes (̵OC₂H₄)̵, où l'un ou plusieurs des atomes d'oxygène peuvent être remplacés par des atomes de soufre :
- les produits de la forme : 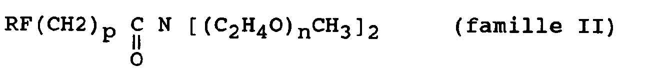 où p est inférieur à 3.

7. Procédé d'obtention de matériaux viscoélastiques selon l'une quelconque des revendications 1 à 6, caractérisé en ce qu'il consiste à mettre en présence le tensioactif fluoré, l'huile fluorée et la quantité nécessaire et suffisante d'eau pour réaliser tout d'abord un prémélange lipide isotrope puis à rajouter progressivement l'eau jusqu'à obtention d'un gel consistant et visqueux.

8. Procédé selon la revendication 7, caractérisé en ce que l'eau est remplacée par un mélange d'eau et d'un solvant miscible à l'eau du type eau-DMSO ou par une solution aqueuse homogène ayant dissous un ou plusieurs solutés.

9. Procédé selon la revendication 8 caractérisé en ce que la solution aqueuse homogène est choisie dans le groupe des solutions ioniques et des milieux de culture cellulaire.

10. Application des matériaux selon l'une quelconque des revendications 1 à 6 dans le domaine de l'optique, des matériaux de remplissage, des cosmétiques, de la pharmacologie, des lubrifiants, des textiles, de la photochimie ou de l'électrodynamique.

## Revendications (Revendications pour l'(les) Etat(s) contractant(s) suivant(s): ES)

1. Procédé de préparation de matériaux isotropes viscoélastiques à base d'eau, de tensioactifs fluorés et d'huiles fluorées ayant l'aspect et la consistance de gels, caractérisés en ce qu'ils renferment de 60 à 98 % en poids d'eau ou d'un mélange eau-solvant miscible à l'eau et que leur structure est une structure compartimentée microcellulaire, présentant des globules d'eau ou de mélange entourés d'une membrane de tensioactif et inclus dans une phase continue huile/tensioactif, cette structure étant imperméable à l'eau et perméable aux composés hydrophobes solubles dans les fluocarbures, dans lequel l'on met en présence le tensioactif fluoré, l'huile Fluorée et la quantité nécessaire et suffisante d'eau pour réaliser tout d'-abord un prémélange lipide isotrope puis on rajoute progressivement l'eau jusqu'à obtention d'un gel consistant et visqueux.

2. Procédé selon la revendication 1, caractérisé en ce que le tensioactif est choisi parmi les tensioactifs non ioniques à chaîne(s) fluorée(s) présentant une hydrophilie caractérisée par une HBL calculée selon la formule de Griffin inférieure à 8.

3. Procédé selon la revendication 2, caractérisé en ce que le tensioactif associe une partie hydrophile comprenant des groupements polyoxyéthylènes-(OC₂H₄)n- avec n > 2, à une partie hydrophobe fluorée comprenant des motifs -CₓF2ₓ-, avec 4 < x < 12.

4. Procédé selon l'une des revendications 1 à 3, caractérisé en ce que les globules d'eau ou de mélange ont une taille moyenne de l'ordre du micron.

5. Procédé selon l'une quelconque des revendications 1 à 4, caractérisé en ce que les tensioactifs fluorés sont des tensioactifs non ioniques à chaîne essentiellement fluorée et que les huiles fluorées sont choisies parmi les carbures totalement ou partiellement fluorés comportant au moins 6 atomes de carbone.

6. Procédé selon la revendication 5, caractérisé en ce que les tensioactifs à chaîne essentiellement fluorée sont choisis parmi :
- les produits de la forme :
RF(CH2)ₚR(OH) (famille I)
où p est inférieur à 3, dans lesquels RF est une chaîne perfluorée,
R désigne un enchaînement de motifs oxyéthylènes (OC₂H₄), où l'un ou plusieurs des atomes d'oxygène peuvent être remplacés par des atomes de soufre :
- les produits de la forme : 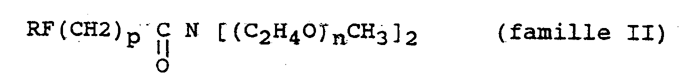 où p est inférieur à 3.

7. Procédé selon l'une des revendications 1 à 6, caractérisé en ce que l'eau est remplacée par un mélange d'eau et d'un solvant miscible à l'eau du type eau-DMSO ou par une solution aqueuse homogène ayant dissous un ou plusieurs solutés.

8. Procédé selon la revendication 7 caractérisé en ce que la.solution aqueuse homogène est choisie dans le groupe des solutions ioniques et des milieux de culture cellulaire.

9. Application des matériaux obtenus par le procédé selon l'une quelconque des revendications 1 à 8 dans le domaine de l'optique, des matériaux de remplissage, des cosmétiques, de la pharmacologie, des lubrifiants, des textiles, de la photochimie ou de l'électrodynamique.

## Patentansprüche (Patentansprüche für folgende(n) Vertragsstaat(en): AT, BE, CH, DE, FR, GB, GR, IT, LI, LU, NL, SE)

1. Isotrope, viskoelastische Materialien auf Basis von Wasser, fluorierten oberflächenaktiven Substanzen und fluorierten Ölen, die das Aussehen und die Konsistenz von Gelen aufweisen, dadurch gekennzeichnet, daß sie 60 bis 98 Gewichts-% Wasser oder einer mit Wasser mischbaren Wasser-Solvens-Mischung enthalten, und daß ihre Struktur eine mikrozellförmige Kompartimentstruktur ist, die Wasser- oder Mischungskügelchen aufweist, welche von einer Membran aus oberflächenaktiver Substanz umschlossen und in eine kontinuierliche Phase aus Öl/oberflächenaktiver Substanz eingeschlossen sind, wobei diese Struktur für Wasser undurchlässig und für hydrophobe Verbindungen, die in den Fluorkohlenstoffen löslich sind, durchlässig ist.

2. Materialien nach Anspruch 1, dadurch gekennzeichnet, daß die oberflächenaktive Substanz unter den nichtionischen oberflächenaktiven Substanzen mit fluorierter/n Kette(n) ausgewählt ist, die eine Hydrophilie aufweisen, welche durch einen nach der Formel von Griffin berechneten HBL-Wert von unter 8 charakterisiert ist.

3. Materialien nach Anspruch 2, dadurch gekennzeichnet, daß die oberflächenaktive Substanz einen hydrophilen Teil, der Polyoxyethylengruppen -(OC₂H₄)ₙ- mit n > 2 aufweist, an einen hydrophoben, fluorierten Teil gebunden enthält, der Einheiten -CₓF₂ₓ- mit 4 < x <12 aufweist.

4. Materialien nach einem der Ansprüche 1 bis 3, dadurch gekennzeichnet, daß die Wasser- oder Mischungskügelchen eine mittlere Größe im Mikrometerbereich besitzen.

5. Materialien nach einem der Ansprüche 1 bis 4, dadurch gekennzeichnet, daß die fluorierten oberflächenaktiven Substanzen nichtionische oberflächenaktive Substanzen mit einer im wesentlichen fluorierten Kette sind, und daß die fluorierten Öle ausgewählt sind unter den vollständig oder teilweise fluorierten Kohlenwasserstoffen mit mindestens 6 Kohlenstoffatomen.

6. Materialien nach Anspruch 5, dadurch gekennzeichnet, daß die oberflächenaktiven Substanzen mit einer im wesentlichen fluorierten Kette ausgewählt sind unter:
- den Produkten mit der Formel:
RF(CH₂)ₚR(OH) (Gruppe I)
worin p weniger als 3 ist, in denen RF eine perfluorierte Kette ist,
R eine Aneinanderreihung von Oxyethyleneinheiten -(OC₂H₄)- bezeichnet, worin ein oder mehrere Sauerstoffatome durch Schwefelatome ersetzt sein können,
- den Produkten mit der Formel: 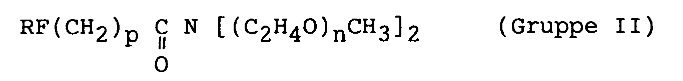 worin p weniger als 3 ist

7. Verfahren zum Herstellen von viskoelastischen Materialien nach einem der Ansprüche 1 bis 6, dadurch gekennzeichnet, daß es aus dem Zusammenbringen der fluorierten oberflächenaktiven Substanz, des fluorierten Öls und derjenigen Menge an Wasser, die notwendig und ausreichend ist, um zuerst eine isotrope Lipid-Vormischung herzustellen, und im Anschluß daran dem allmählichen Hinzufügen des Wassers bis zur Gewinnung eines konsistenten und viskosen Gels besteht.

8. Verfahren nach Anspruch 7, dadurch gekennzeichnet, daß das Wasser durch eine Mischung aus Wasser und einem mit Wasser mischbaren Solvens von der Art Wasser-DMSO oder durch eine wäßrige, homogene Lösung ersetzt ist, in der ein oder mehrere Stoffe gelöst vorliegen.

9. Verfahren nach Anspruch 8, dadurch gekennzeichnet, daß die wäßrige, homogene Lösung aus der Gruppe der Ionen enthaltenden Lösungen und der Zellkulturmedien ausgewählt ist.

10. Verwendung der Materialien nach einem der Ansprüche 1 bis 6 auf dem Gebiet der Optik, der Füllmaterialien, der Kosmetika, der Pharmakologie, der Schmiermittel, der Textilien, der Photochemie oder der Elektrodynamik.

## Patentansprüche (Patentansprüche für folgende(n) Vertragsstaat(en): ES)

1. Verfahren zum Herstellen von isotropen, viskoelastischen Materialien auf Basis von Wasser, fluorierten oberflächenaktiven Substanzen und fluorierten Ölen, die das Aussehen und die Konsistenz von Gelen aufweisen, dadurch gekennzeichnet, daß sie 60 bis 98 Gewichts-% Wasser oder einer mit Wasser mischbaren Wasser-Solvens-Mischung enthalten, und daß ihre Struktur eine mikrozellförmige Kompartimentstruktur ist, die Wasser- oder Mischungskügelchen aufweist, welche von einer Membran aus oberflächenaktiver Substanz umschlossen und in eine kontinuierliche Phase aus Öl/oberflächenaktiver Substanz eingeschlossen sind, wobei diese Struktur für Wasser undurchlässig und für hydrophobe Verbindungen, die in den Fluorkohlenstoffen löslich sind, durchlässig ist, worin man die fluorierte oberflächenaktive Substanz, das fluorierte Öl und diejenige Menge an Wasser, die notwendig und ausreichend ist, um zuerst eine isotrope Lipid-Vormischung herzustellen, zusammenbringt und im Anschluß daran nach und nach das Wasser bis zur Gewinnung eines konsistenten und viskosen Gels hinzufügt.

2. Verfahren nach Anspruch 1, dadurch gekennzeichnet, daß die oberflächenaktive Substanz unter den nichtionischen oberflächenaktiven Substanzen mit fluorierter/n Kette(n) ausgewählt ist, die eine Hydrophilie aufweisen, welche durch einen nach der Formel von Griffin berechneten HBL-Wert von unter 8 charakterisiert ist.

3. Verfahren nach Anspruch 2, dadurch gekennzeichnet, daß die oberflächenaktive Substanz einen hydrophilen Teil, der Polyoxyethylengruppen -(OC₂H₄)ₙ- mit n > 2 aufweist, an einen hydrophoben, fluorierten Teil gebunden enthält, der Einheiten -CₓF₂ₓ- mit 4 < x <12 aufweist.

4. Verfahren nach einem der Ansprüche 1 bis 3, dadurch gekennzeichnet, daß die Wasser- oder Mischungskügelchen eine mittlere Größe im Mikrometerbereich besitzen.

5. Verfahren nach einem der Ansprüche 1 bis 4, dadurch gekennzeichnet, daß die fluorierten oberflächenaktiven Substanzen nichtionische oberflächenaktive Substanzen mit einer im wesentlichen fluorierten Kette sind, und daß die fluorierten Öle ausgewählt sind unter den vollständig oder teilweise fluorierten Kohlenwasserstoffen mit mindestens 6 Kohlenstoffatomen.

6. Verfahren nach Anspruch 5, dadurch gekennzeichnet, daß die oberflächenaktiven Substanzen mit einer im wesentlichen fluorierten Kette ausgewählt sind unter:
- den Produkten mit der Formel:
RF(CH₂)ₚR(OH) (Gruppe I)
worin p weniger als 3 ist, in denen RF eine perfluorierte Kette ist,
R eine Aneinanderreihung von Oxyethyleneinheiten (OC₂H₄) bezeichnet, worin ein oder mehrere Sauerstoffatome durch Schwefelatome ersetzt sein können,
- den Produkten mit der Formel: 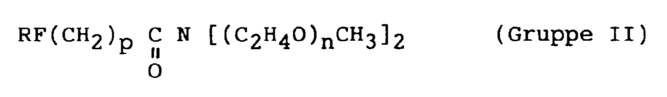 worin p weniger als 3 ist.

7. Verfahren nach einem der Ansprüche 1 bis 6, dadurch gekennzeichnet, daß das Wasser durch eine Mischung aus Wasser und einem mit Wasser mischbaren Solvens von der Art Wasser-DMSO oder durch eine wäßrige, homogene Lösung ersetzt ist, in der ein oder mehrere Stoffe gelöst vorliegen.

8. Verfahren nach Anspruch 7, dadurch gekennzeichnet, daß die wäßrige, homogene Lösung unter der Gruppe der Ionen enthaltenden Lösungen und der Zellkulturmedien ausgewählt ist.

9. Verwendung der Materialien, die nach dem Verfahren gemäß einem der Ansprüche 1 bis 8 erhalten wurden, auf dem Gebiet der Optik, der Füllmaterialien, der Kosmetika, der Pharmakologie, der Schmiermittel, der Textilien, der Photochemie oder der Elektrodynamik.

## Claims (Claims for the following Contracting State(s): AT, BE, CH, DE, FR, GB, GR, IT, LI, LU, NL, SE)

1. Viscoelastic isotropic materials based on water, fluorinate surfactants and fluorinated oils which have the appearance and-the consistency of gels, characterised in that they contain from 60 to 98 % by weight of water or of a water-miscible water/solvent mixture and that their structure is a microcellular compartmented structure exhibiting globules of water or of mixture which are surrounded by a surfactant membrane and included in a continuous oil/surfactant phase, this structure being impervious to water and permeable to hydrophobic compounds which are soluble in fluorocarbons.

2. Materials according to Claim 1, characterised in that the surfactant is chosen from nonionic surfactants containing a fluorinated chain or chains, exhibiting a hydrophilicity characterised by an HLB calculated according to the Griffin formula which is lower than 8.

3. Materials according to Claim 2, characterised in that the surfactant combines a hydrophilic part containing polyoxyethylene groups -(OC₂H₄)ₙ- with n > 2, with a fluorinated hydrophobic part containing -CₓF₂ₓ- units, with 4 < x < 12.

4. Materials according to one of Claims 1 to 3, characterised in that the globules of water or of mixture have a mean size of the order of a micron.

5. Materials according to any one of Claims 1 to 4, characterised in that the fluorinated surfactants are nonionic surfactants containing an essentially fluorinated chain and that the fluorinated oils are chosen from wholly or partially fluorinated carbon compounds containing at least 6 carbon atoms.

6. Materials according to Claim 5, characterised in that the surfactants containing an essentially fluorinated chain are chosen from:
- the products of the form:
RF(CH₂)ₚR(OH) (class I)
where p is lower than 3, in which RF is a perfluorinated chain,
R denotes a chain sequence of oxyethylene units (̵OC₂H₄)̵, where one or more of the oxygen atoms may be replaced by sulphur atoms, and
- the products of the form: 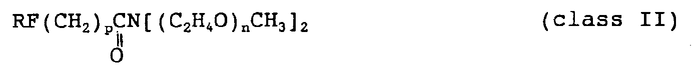 where p is lower than 3.

7. Process for obtaining viscoelastic materials according to any one of Claims 1 to 6, characterised in that it consists in bringing together the fluorinated surfactant, the fluorinated oil and the necessary and sufficient quantity of water to produce first of all an isotropic lipid premix and then in progressively adding water until a stable and viscous gel is obtained.

8. Process according to Claim 7, characterised in that the water is replaced by a mixture of water and of a water-soluble solvent of the water/DMSO type or by a homogeneous aqueous solution which has dissolved one or more solutes.

9. Process according to Claim 8, characterised in that the homogeneous aqueous solution is chosen from the group of ionic solutions and cell culture media.

10. Application of the materials according to any one of Claim 1 to 6 in the field of optics, of filling materials, of cosmetics, of pharmacology, of lubricants, of textiles, of photochemistry or of electrodynamics.

## Claims (Claims for the following Contracting State(s): ES)

1. Process for the preparation of viscoelastic isotropic materials based on water, fluorinated surfactants and fluorinated oils which have the appearance and the consistency of gels, characterised in that they contain from 60 to 98 % by weight of water or of a water-miscible water/solvent mixture and that their structure is a microcellular compartmented structure exhibiting globules of water or of mixture which are surrounded by a surfactant membrane and included in a continuous oil/surfactant phase, this structure being impervious to water and permeable to the hydrophobic compounds which are soluble in fluorocarbons, in which the fluorinated surfactant, the fluorinated oil and the necessary and sufficient quantity of water are brought together to produce first of all an isotropic lipid premix and water is then progressively added until a stable and viscous gel is obtained.

2. Process according to Claim 1, characterised in that the surfactant is chosen from nonionic surfactants containing a fluorinated chain or chains, exhibiting a hydrophilicity characterised by an HLB calculated according to the Griffin formula which is lower than 8.

3. Process according to Claim 2, characterised in that the surfactant combines a hydrophilic part containing polyoxyethylene groups -(OC₂H₄)ₙ- with n > 2, with a fluorinated hydrophobic part containing -CₓF₂ₓ- units, with 4 < x < 12.

4. Process according to one of Claims 1 to 3, characterised in that the globules of water or of mixture have a mean size of the order of a micron.

5. Process according to any one of Claims 1 to 4, characterised in that the fluorinated surfactants are nonionic surfactants containing an essentially fluorinated chain and that the fluorinated oils are chosen from wholly or partially fluorinated carbon compounds containing at least 6 carbon atoms.

6. Process according to Claim 5, characterised in that the surfactants containing an essentially fluorinated chain are chosen from:
- the products of the form:
RF(CH₂)ₚR(OH) (class I)
where p is lower than 3, in which RF is a perfluorinated chain,
R denotes a chain sequence of oxyethylene units (OC₂H₄), where one or more of the oxygen atoms may be replaced by sulphur atoms, and
- the products of the form: 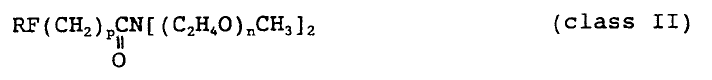 where p is lower than 3.

7. Process according to one of Claims 1 to 6, characterised in that the water is replaced by a mixture of water and of a water-soluble solvent of the water/DMSO type or by a homogeneous aqueous solution which has dissolved one or more solutes.

8. Process according to Claim 7, characterised in that the homogeneous aqueous solution is chosen from the group of ionic solutions and cell culture media.

9. Application of the materials obtained by the process according to any one of Claims 1 to 8 in the field of optics, of filling materials, of cosmetics, of pharmacology, of lubricants, of textiles, of photohemistry or of electrodynamics.
